(19) Europäisches Patentamt
European Patent Office
Office européen des brevets

(11) Publication number: **0 170 265**
**B1**

(12) # EUROPEAN PATENT SPECIFICATION

(45) Date of publication of patent specification: **16.06.87**

(51) Int. Cl.⁴: **C 07 C 43/205**, A 61 K 7/46

(21) Application number: **85109589.3**

(22) Date of filing: **30.07.85**

(54) **4-Alkylphenyl-2'-alkoxyethyl ethers and fragrance compositions containing same.**

(30) Priority: **01.08.84 US 636772**

(43) Date of publication of application:
**05.02.86 Bulletin 86/06**

(45) Publication of the grant of the patent:
**16.06.87 Bulletin 87/25**

(84) Designated contracting states:
**BE CH DE FR GB IT LI NL SE**

(56) References cited:
**US-A- 2 451 149**
**US-A- 4 404 407**
**CHEMICAL ABSTRACTS, vol. 54, no. 1, January 10, 1960, Columbus, Ohio, USA. A. A. AROYAN: "Chloromethylation of some phenol ethers", column 365e**

(73) Proprietor: **NATIONAL DISTILLERS AND CHEMICAL CORPORATION**
**99 Park Avenue**
**New York, NY 10016 (US)**

(72) Inventor: **Harris, Eugene G.**
**7019 Sprucehill Circle**
**West Chester Ohio (US)**

(74) Representative: **Patentanwälte Grünecker, Kinkeldey, Stockmair & Partner**
**Maximilianstrasse 58**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue St-Denis, 75001 Paris, France

**Description**

This invention relates to fragrance compositions and more particularly to fragrance compositions containing novel 4-alkylphenyl-2'-alkoxyethyl ether compounds.

While many natural perfume chemicals, such as essential oils, like, oil of rose and oil of cloves, and animal secretions, such as musk, are known, a large number of synthetic odoriferous chemicals possessing aroma characteristics have been developed. Synthetic aroma chemicals have added a new dimension to the art of perfuming, since these synthetics are usually stable compounds and are relatively inexpensive, as compared with the natural perfume chemicals. For example, ethylene glycol monoaryl ethers of the general formula :

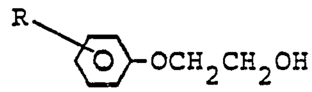

are known fragrance compounds having a mild rose odor and are useful for food, comestic and pharmaceutical applications. See, for example, U.S. Patent Nos. 1,881,200, 2,451,149 and 4,404,407. Moreover, synthetics lend themselves more easily to manipulation than natural perfume chemicals since natural perfume chemicals are usually a complex mixture of substances which defy chemical analysis. Accordingly, for these and other reasons, there is a great desire in the art of fragrance chemistry for new compounds possessing specific characteristic aromas.

Japanese Patent No. 53-18524 discloses compounds of the formula :

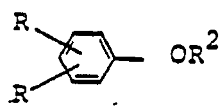

wherein $R^2$ is $CH_2OCH_3$ or H and R and R' are H, alkyl or halo to have bactericidal activity. Chemical Abstracts 54 : 365 (1960) discloses, inter alia, compounds of the formulae :

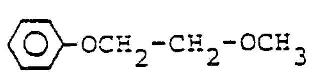 and 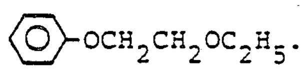

These references do not disclose these compounds or derivatives thereof to have fragrant qualities useful for additives in fragrant compositions.

The present invention relates to novel aroma compounds having the general formula :

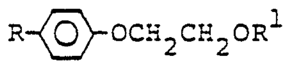

wherein R is lower alkyl having from 1 to 2 carbon atoms and $R^1$ is lower alkyl having from 1 to 2 carbon atoms.

The present invention also relates to a process for preparing a compound of the formula :

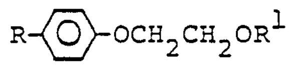

wherein R and $R^1$ are each independently lower alkyl having from 1 to 2 carbon atoms which comprises reacting p-alkylphenols with haloethyl alkyl ethers in the presence of a base or alternatively reacting 4-alkylphenoxyethanol with dialkylsufate, or an alkyl halide, in the presence of a base, at a temperature sufficient to promote said reaction.

These compounds are characterized with a pleasant natural green foliage note and are employed herein as fragrant additives in fragrance compositions.

In accordance with the present invention, it has been found that certain novel 2'-alkoxyethoxy-4-alkylbenzenes have a very pleasant natural green foliage note very similar to natural privet. More particularly, the novel compounds of the present invention which possess this desirable property have the general formula :

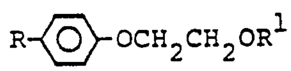

wherein R is methyl or methyl, and $R^1$ is also lower alkyl of from 1 to 2 carbon atoms, i. e., methyl or ethyl. Preferably R and $R^1$ are methyl. Thus, a most preferred compound within the scope of the present

invention is 4-methylphenyl-2'-methoxyethyl ether.

The novel compounds of the present invention can be prepared by a variety of techniques. For example, one method involves the reaction of p-alkylphenols, e. g., of p-cresol, with haloethylalkyl ethers, e. g., chloroethylmethyl ether, in the presence of alkali hydroxide, such as sodium hydroxide. Another technique involves reacting p-alkylphenols, e. g., p-cresol, with ethylene oxide in the presence of potassium hydroxide and sodium borohydride. The 4-alkylphenoxyethanol is then reacted with a dialkylsulfate, such as dimethylsulfate, in the presence of sodium hydroxide and tetrabutylammonium iodide or methyl chloride in the presence of sodium or potassium hydroxide at 60-100 °C. The odor characteristics of the desired compounds of the present invention are independent of the synthetic route.

As a result of their pleasing aroma, the novel compounds of the present invention are useful as fragrances in the preparation and formulation of fragrance compositions, such as perfumes, and perfumed products. Thus, the term fragrance compositions is intended herein to mean products such as perfume and perfume products such as soaps, washing agents, dishwashing and other detergents, air fresheners and room sprays, toliet preparations, ·pomanders, candles, comestics, such as creams, colognes, pre- and after-shaving lotions, talcum powder, hair care agents, body deodorants and antiperspirants. The novel compounds of this invention can be utilized as the primary fragrance in many such compositions or may be combined with other compatible fragrances. The compounds of this invention have a better stability in acidic cleaners and oxidative cleaners (bleaches) than the materials that are now available and have a green odor.

In most applications, the compounds of this invention can be used in amounts of from about 0.01 to about 10 % by weight of the fragrance composition.

Accordingly, the following examples are presented by way of illustration and not by way of limitation so that those skilled in the art may better understand how to practice the invention.

## Example 1

Preparation of 4-methylphenyl-2'-methoxyethyl ether

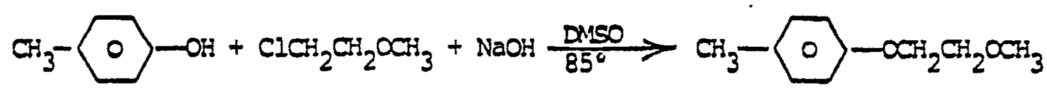


113.9 gms. (1.055 moles, 110.2 mls.) of p-cresol 84.4 gms. (2.11 moles) of NaOH and 319 mls. of dimethylsulfoxide (DMSO) are charged to a 2-liter flask fitted with a mechanical stirrer, condenser, thermometer and addition funnel. The mixture is heated to 85 °C for 1 hour. 200 gms. (2.11 moles, 193 mls.) of chloroethylmethyl ether are then added to the reaction mixture over 2 hours with no heat applied until the end of the addition at which time the reaction mixture is heated at 85 °C for 3 hours. A sample is removed for GC analysis and shows 100 % desired product. Sufficient water is then added to the reaction mixture to dissolve salts. Organic and aqueous layers separated ; then the organics are taken up in ether and the water layer is extracted with ether.

The extracts are combined and dried over $MgSO_4$. Filtration and removal of ether solvent results in 184 gms. of product.

Distilled via 6" Vigreaux column. Gms = 167 gms.

B.P. = 70 °C at 0.6 mmHg
GC = 100 %
IR = C—O—C stretch at 1 240 $cm^{-1}$
NMR = confirms product structure

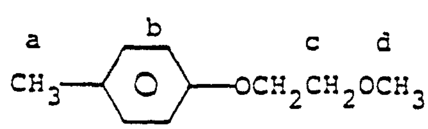

| δ | H |
|---|---|
| 2.24 singlet | a |
| 6.76-7.2 multiplet | b |
| 3.55-4.25 multiplet | c |
| 3.4 singlet | d |

Theoretical yield = 1.055 moles = 175 gms.
Actual Yield = 167 gms. = 1.006 moles
Percent yield = 95.4 %

## Example 2

This example illustrates another technique to prepare 4-methylphenyl-2'-methoxyethyl ether.

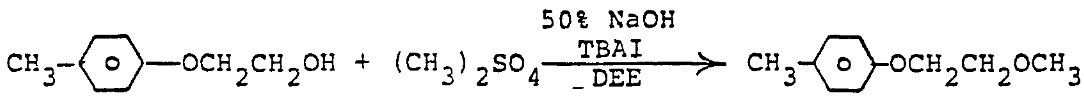


50 gms. (0.33 moles) of 4-methylphenoxy ethanol and 2.43 gms. (0.0066 moles, 2 mole%) of tetrabutylammonium iodide (TBAI) are charged to a 500 ml. flask fitted with mechanical stirrer, condenser, addition funnel and $N_2$ inlet. 100 mls. of diethylether (DEE) are added, since the starting alcohol is a solid.

66 gms. (1.65 moles) of sodium hydroxide (as a 50 % aqueous solution) are then added to the flask over 15 minutes. The mixture is then stirred for 1/2 hour becoming very thick and white. 108 gms. (0.86 moles = 81 mls.) of dimethyl sulfate are added over 1 hour, with the reaction mixture becoming less thick as addition nears completion. The mixture is stirred for two hours.

The resultant product is rinsed into 150 mls. of distilled water. Layers separate and the organic layer is washed three times with water. More diethyl ether (DEE) is added and organics are dried over $MgSO_4$. Filtration and removal of solvent affords 50 gms. of material whose GC analysis shows it to b 98.3 % product.

IR — very little OH stretch at 3 500 $cm^{-1}$

C—O—C, aromatic 1 250 $cm^{-1}$
C—O—C, aliphatic 1 135 $gm^{-1}$ confirms product structure

Actual yield = 50 gms. (0.983) = 49.2 gms. = 0.30 moles

Theoretical yield = 0.33 moles = 54.8 gms.

% yield = 90.9 %

NMR

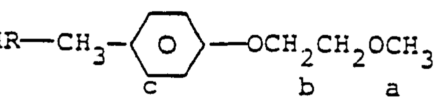

| δ | H |
|---|---|
| 3.4 S | a |
| 3.55-4.25 M | b |
| 6.7-7.2 M | c |
| 2.25 S | d |

B.P. = 65 °C at 0.25 mmHg (100 %)

The 4-methylphenyl-2'-methoxyethyl ether obtained via the above-described procedures has a natural leaf green odor. The complex odor properties include, but are not limited to, those found in phenylacetaldehyde dimethylacetal and 3-phenylpropyl alcohol. Nuances of lilac, Elang and rose are evident in the odor profile.

## Example 3

This example illustrates the preparation of fragranced soap bars.

Four large batches of soap are run through the Mazzoni process. For each run, 1 800 gms. of a commercial soap stock and 1 % by weight of fragrance chemical are used. Water is added during processing to provide the necessary plasticity. The 4-methylphenyl-2'-methoxyethyl ether is added to the soap stock and thoroughly blended in before the soap stock is extruded in a tubular form. Fragranced soap bars are then stamped from sections of the extruded tube.

The bars prepared in this manner had odor properties similar to neat 4-methylphenyl-2'-methoxyethyl ether.

## Example 4

A sample of 4-methylphenyl-2'-methoxyethyl ether is mixed with a household bleach solution, of 5.25 % NaOCl by weight, and stored in darkness for two days. A control bleach solution is also stored in darkness for two days.

The amount of NaOCl in each sample is determined by iodometric analysis. Each is run two times for 2 days and 6 days.

Results show the 4-methylphenyl-2'-methoxyethyl ether to have no effect on the degradation of the bleach within the experimental limitations of analysis and suffered no odor change.

The sample of bleach that included the 4-methylphenyl-2'-methoxyethyl ether had a more pleasant, less chemical odor than the unfragranced bleach sample.

Example 5

4-methylphenyl-2′-methoxyethyl ether is adsorbed on a cosmetic grade talc at a 0.1 weight percent level. More of the natural green rose character of the product is evident when placed on the talc. A small portion of the fragranced talc is subjected to ultraviolet radiation for twenty-four hours and there is no noticeable discoloration or change in odor characteristics. Another portion of the fragranced talc is stored at 45 °C for two weeks in a closed container without discoloration or change in odor characteristics.

Example 6

4-methylphenyl-2′-methoxyethyl ether is added to an herbal perfume base at a 10 weight percent level to enhance the natural green characteristics of the formulation. Incorporation of the perfume base in a shampoo and bar soap provides products having a very natural herbal, fresh odor with good persistence.

Example 7

A toilet bowl cleaner is prepared in accordance with the following information :

| | Parts by Weight |
|---|---|
| 20° Baume Hydrochloric Acid | 120 |
| Tricaprylylmethylammonium Chloride | 4 |
| Imidazoline of Coconut Fatty Acids | 4 |
| Nonylphenoxy Polyethoxyethanol | 8 |
| Water | 264 |
| | 400 |

All of the ingredients are combined and thoroughly mixed. An aliquot of the solution is removed and 1 weight percent 4-methylphenyl-2′-methoxyethyl ether added thereto. The resulting fragranced toilet bowl cleaner preparation has a natural, fresh scent.

Example 8

A fragranced detergent is prepared by adsorbing 0.01 weight percent 4-methylphenyl-2′-methoxyethyl ether on a commercially available unfragranced detergent. The fragranced detergent thus prepared has a pleasant fresh odor. When the fragranced detergent is mixed into warm water, a pleasant fresh green odor is noted.

Example 9

A liquid soap is prepared in accordance with the following formulation :

| | |
|---|---|
| Emersal® 6400 Sodium Laury Sulfate | 30.0 |
| Emid® 6511 Lauramide DEA | 6.0 |
| Lanoquat® 1756 Lanolin Quaternary. | 1.0 |
| Emerest® 2350 Glycol Stearate | 1.0 |
| Emersol® 132 Stearic Acid | 0.5 |
| Triethanolamine | 0.3 |
| Emeressence® 1160 Rose Ether™ Phenoxyethanol | 1.0 |
| Deionized Water | 60.2 |
| | 100.0 |

The ingredients are combined and heated slowly to 75 °C until all of the components melt. The mixture is then cooled to 40 °C with agitation and 0.5 weight percent 4-methylphenyl-2′-methoxyethyl ether blended into the formulation. The resulting liquid soap preparation has a very natural, herbal odor.

Example 10

A mild shampoo base is prepared in accordance with the following recipe :

| | |
|---|---|
| Emersal® 6455 Sodium Laureth Sulfate | 20.0 |
| Emery® 5320 Laureth Sulfosuccinate | 10.0 |
| Emid® 6515 Cocamide DEA | 5.0 |

| | |
|---|---|
| Emery® 5412 Cocoamphoglycinate | 4.0 |
| Emerescence® 1160 Rose Ether™ Phenoxyethanol | 0.7 |
| Deionized Water | 60.3 |
| | 100.0 |

The ingredients are combined and heated with agitation until a homogeneous blend is obtained. Viscosity and pH are then adjusted by the addition of small increments of sodium chloride and citric acid, respectively. One-half weight percent 4-methylphenyl-2'-methoxyethyl ether is then blended into the shampoo base. The resulting shampoo has a very pleasant natural, herbal odor.

Obviously, other modifications and variations of the present invention are possible in light of the above teachings. It is therefore to be understood that changes may be made in the particular embodiments of this invention which are within the full intended scope of the invention as defined by the appended claims.

## Claims

1. A compound having the structural formula

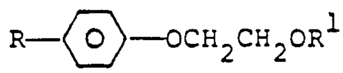

wherein

R is lower alkyl having from 1 to 2 carbon atoms and

$R^1$ is lower alkyl having from 1 to 2 carbon atoms.

2. The compound of claim 1 wherein R is methyl and $R^1$ is methyl.

3. A fragrance composition having incorporated therein an odoriferous amount of a compound defined in claim 1.

4. A fragrance composition according to claim 3, characterised by a compound as defined in claim 2.

5. A process for providing a composition with a fragrance composition characterised by incorporating therein an odoriferous amount of the compound defined in claim 1.

6. A process according to claim 5, characterised by the compound defined in claim 2.

## Patentansprüche

1. Verbindung mit der Strukturformel

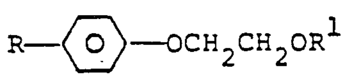

worin

R Niederalkyl mit 1 bis 2 Kohlenstoffatomen und

$R^1$ Niederalkyl mit 1 bis 2 Kohlenstoffatomen bedeuten.

2. Verbindung nach Anspruch 1, wobei R Methyl und $R^1$ Methyl bedeuten.

3. Duftstoffzusammensetzung, die eine riechende Menge einer in Anspruch 1 definierten Verbindung enthält.

4. Duftstoffzusammensetzung nach Anspruch 3, gekennzeichnet durch eine in Anspruch 2 definierte Verbindung.

5. Verfahren zum Vorsehen einer Zusammensetzung mit einer Duftstoffzusammensetzung, gekennzeichnet durch Einarbeiten hierin einer riechenden Menge der in Anspruch 1 definierten Verbindung.

6. Verfahren nach Anspruch 5, gekennzeichnet durch die in Anspruch 2 definierte Verbindung.

## Revendications

1. Composé représenté par la formule structurale :

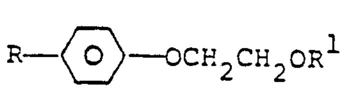

dans laquelle :

R représente un groupe alkyle inférieur possédant 1 ou 2 atomes de carbone ; et,

$R^1$ représente un groupe alkyle inférieur possédant 1 ou 2 atomes de carbone.

2. Composé selon la revendication 1, dans lequel R représente un groupe méthyle et $R^1$ représente un groupe méthyle.

3. Composition parfumante dans laquelle est incorporée une quantité parfumante d'un composé tel que défini à la revendication 1.

4. Composition parfumante selon la revendication 3, caractérisée par un composé tel que défini à la revendication 2.

5. Procédé pour rendre parfumée une composition, caractérisé par le fait qu'on incorpore à ladite composition une quantité odorante du composé tel que défini à la revendication 1.

6. Procédé selon la revendication 5, caractérisé par le composé tel que défini à la revendication 2.